# EUROPEAN PATENT SPECIFICATION

(11) **EP 2 512 377 B1**
(45) Date of publication and mention of the grant of the patent: **15.07.2015**
(21) Application number: 10759745.2
(22) Date of filing: 24.05.2010
(51) Int. Cl.: A61F 2/28, A61F 2/30

(54) **A BIOMATERIAL**
BIOMATERIAL
BIOMATÉRIAU

(30) Priority: 25.05.2009 TR 200904029
(43) Date of publication of application: 24.10.2012
(73) Proprietor: Aydin, Halil Murat, 06660 Ankara (TR); Ozgurbuz, Levent Mete, 06550 Ankara (TR)
(72) Inventor: Aydin, Halil Murat, 06660 Ankara (TR); Ozgurbuz, Levent Mete, 06550 Ankara (TR)
(74) Representative: Dericioglu Kurt, Ekin
(86) International application number: PCT/TR2010/000101
(87) International publication number: WO 2010/144065

(56) References cited:
- WO-A1-2008/016862
- WO-A1-2009/111069
- WO-A2-2006/083946

## Description

### Field of the invention

This invention is a biomaterial used for the treatment of osteochondral bone defects and particularly defects of the hyaline cartilage covering bone.

### Background of the invention

One of the biggest medical problems of our day is the loss of bone and cartilage tissue due to factors such as aging, disease, cysts or tumour resectioning. To solve this problem, the most important and common approach is trying to cover the defect area with a suitable biomaterial.

Biomaterial is the general name given to materials and devices which contact the living system within the body (*in vivo*) or outside the body (*in vitro*) for a certain time in order to perform a certain function (Levin, 1968). Biomaterials are generally categorized in two groups, namely natural and synthetic.

Said natural biomaterials are usually defined as graft materials and depending on the size of defect area, they can be obtained from different donor (allograft) or from different types (xenograft) after being subjected to various chemical reactions as well as they can be obtained from the patient himself (autograft).

The best treatment method of these would be to place the graft material taken from the patient's own healthy tissue to the defect area. However, it is not possible to find a sufficient size of tissue from the patient in cases where tissue losses in the defect area is large. In the other two treatment methods the biggest problem is the immune response of the recipient. The differences between the donors create an unstable characteristic for the graft and they cannot be completely used in place of the new bone tissue as in autografts. Moreover, the cost of the processes which said allograft and xenograft materials are subjected to before being used in the body are considerably expensive. Besides, the performed procedures decrease both the mechanical and biological resistance of the material. It also doesn't eliminate the risk of infectious diseases and immune responses despite all these procedures.

Another one of the applications directed to treatment in hard tissue repair is the use of non - natural (synthetic) biomaterials. There are various known biomaterials such as various screws, rods, and tissue structures etc. used especially in the treatment of bone and cartilage tissue loss.

In the existing technology section of WO2009111069 international patent document, there is a mention of a multi-component device used in the treatment of articular cartilage tissue. With a head and body component, this device is made from materials that are biocompatible with bone and cartilage tissue. The description in the document is of having the head unit fixed on to the body unit. This is not a desirable feature in terms of implantation and treatment flexibility.

Synthetic materials such as porous foams or polymers which are manufactured from biocompatible materials placed into the bone and cartilage tissue layers are described in US patent applications US4186448, US5607474 and US5716413.

Another screw developed for the fixation of the bone tissue together is disclosed in nternational patent application WO0117447.

In United States patent application US2007185585 teaches a scaffold which has at least one channel to renew the bone and cartilage tissue losses, which is made of a porous polymeric material and wherein inside the said channel there is autogenic, allogenic and cultured tissue or a combination thereof.

The closest prior art is described in WO 2008/016862.

However, the materials used in the abovementioned applications especially fails to form an interface that provides contact with the surrounding tissue at the desired level and therefore can not allow vascularization sufficiently. Consequently, tissue renewal and recovery becomes lower than expected. Furthermore, no solution could be found for the necrosis problem caused by the biomaterials used today.

### Brief description of the invention

The object of the present invention is to provide a biocompatible biomaterial which increases the contact surface with the surrounding tissues in order to be used in the treatment of osteochondral bone defects.

Another object of the invention is to provide a biomaterial which provides a high level of vascularization, thus allowing transfer of nutrients and the oxygen to the medium and allowing removal of metabolites and toxic materials from the medium, which makes high level of recovery possible by minimizing formation of necrosis in the tissue.

Another one of the objects of the present invention is to provide a biomaterial which can degrade faster compared to present biomaterials.

Another object of the present invention is to provide a biomaterial which is easy to process and easy to manufacture and apply.

### Detailed description of the invention

The biomaterial realized in order to achieve the objects of the present invention is illustrated in the attached figures in which:

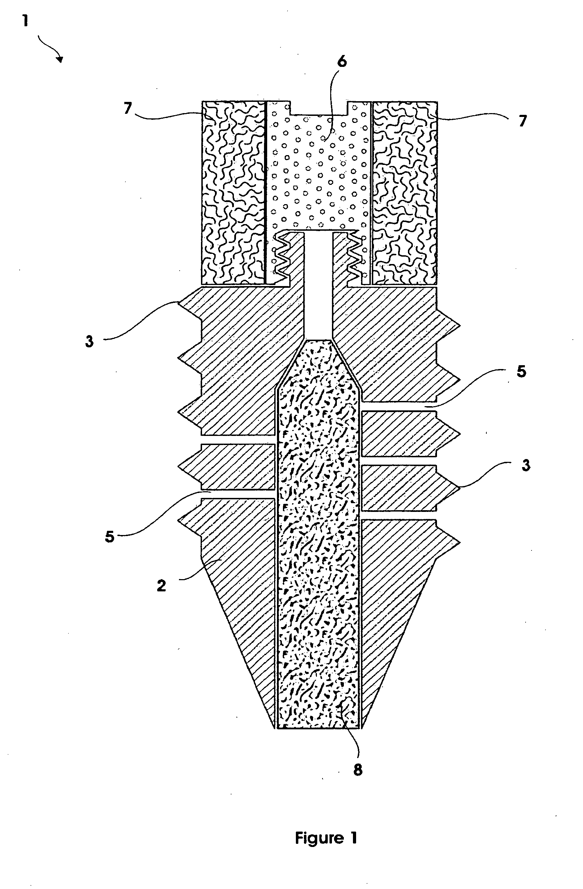

Figure 1 - A front view of the biomaterial of the invention.

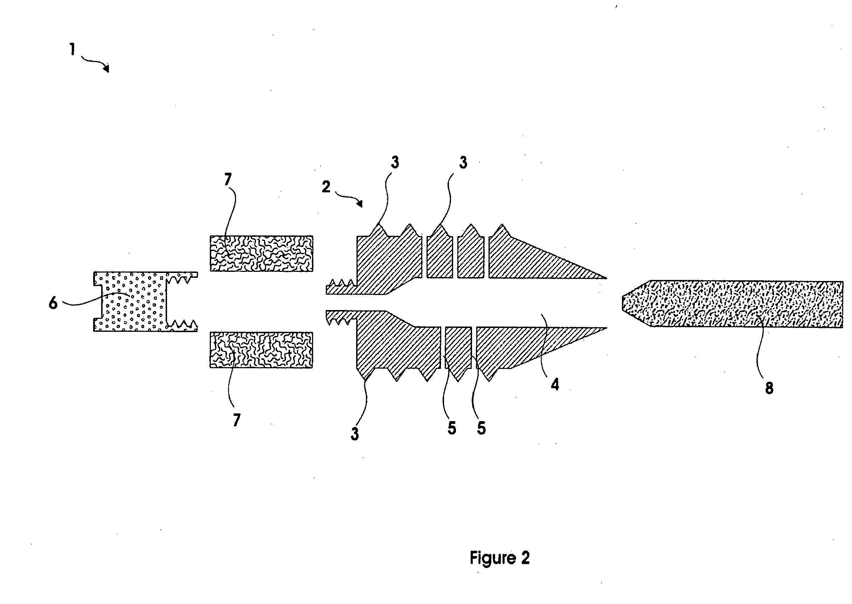

Figure 2 - An exploded side view of the biomaterial of the invention.

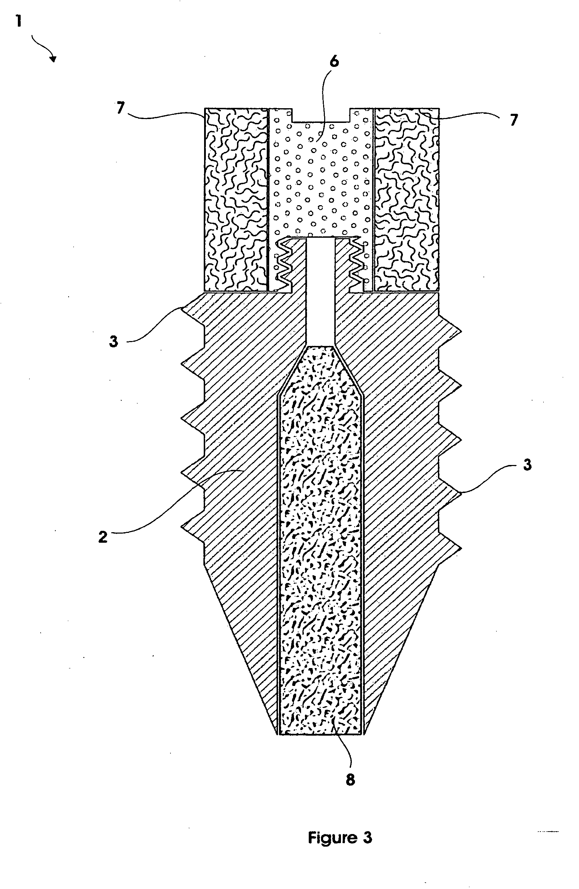

Figure 3 - A view of an embodiment of the biomaterial of the invention.

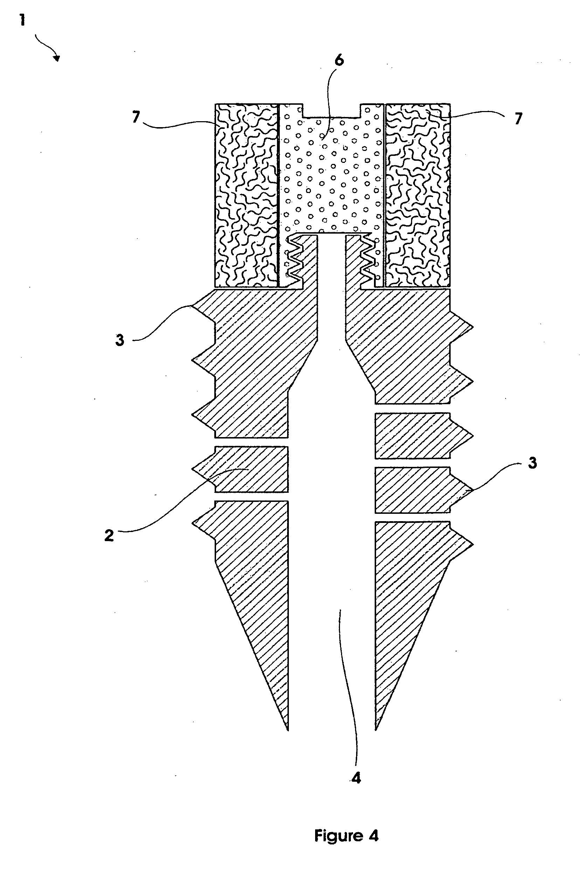

Figure 4 - A view of another embodiment of the biomaterial of the invention.

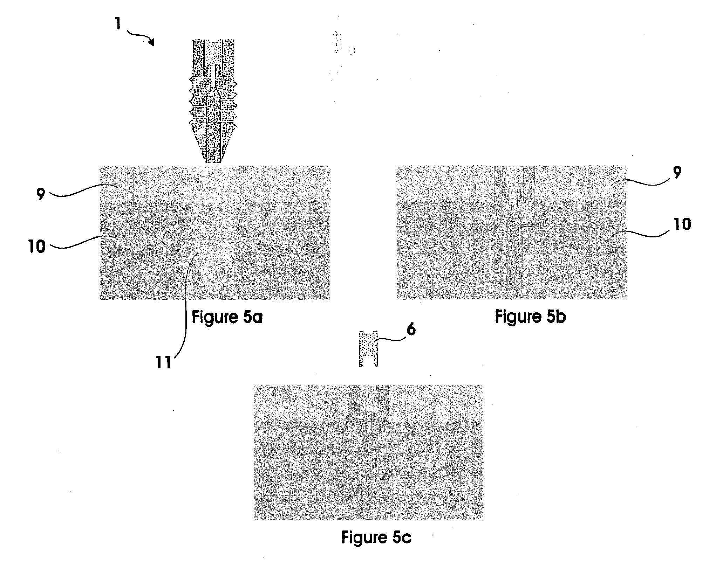

Figure 5a - A view of the biomaterial of the invention before application.
Figure 5b - A view of the biomaterial of the invention in the defect area.
Figure 5c - An application view of the head part of the biomaterial of the invention.

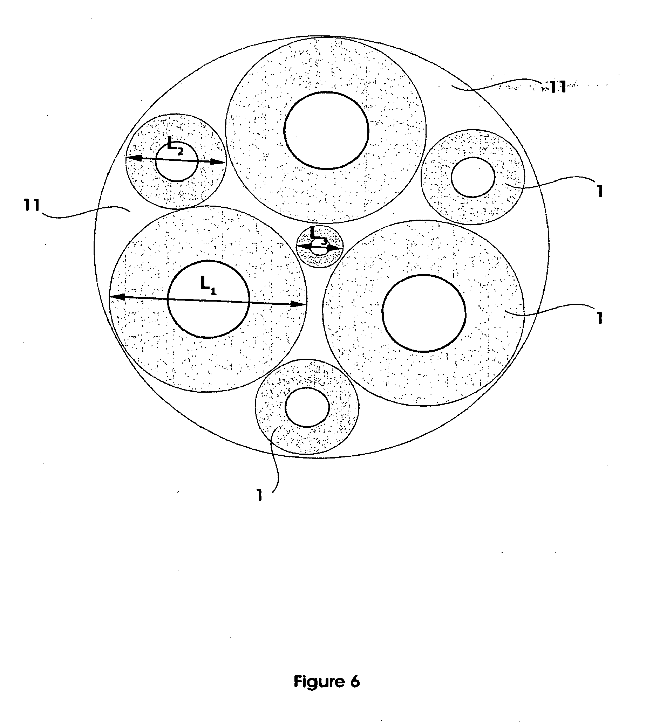

Figure 6 - A top view of an embodiment of the biomaterial of the invention.

The parts in these figures are individually given reference numbers which are stated below:
1. Biomaterial
2. Body
3. Groove
4. Space
5. Channel
6. Header
7. Felt
8. Scaffold
9. Cartilage tissue
10. Bone tissue
11. Defect area

The biomaterial (1) of the invention comprises a body (2) manufactured from a biocompatible material, grooves (3) that allow advancing in the defect area (11) on the body (2), a space (4) which increases the contact area with the surrounding tissue in the body (2) and at least one channel (5) which opens to the space (4) and allows the blood and oxygen to be transferred into the medium and allows metabolites and toxic materials to be removed from the medium, characterized in that it comprises a felt (7) part which is affixed to the upper part of the body (2) by using biocompatible glues known in the art after being prepared in layers and then being cut in a suitable geometry by using a drill or similar device, at least one scaffold (8) which is placed inside the space (4) and which will allow new bone tissue (10) formation and a header (6) which is removed after the material (1) is placed into the defect area (11) in the body.

The body (2) is manufactured from materials selected from alpha hydroxy acids (polyglycolide (PGA), poly(L - lactate), poly(D, L - lactate), poly(ε - caprolactone), poly(trimethylene carbonate), poly(ethylene oxide) (PEO), polyhydroxy butyrate (PHA), poly(β - hydroxybutyrate) (PHB), poly(β - hydroxyvalerate) (PHVA), poly(p-dioxane) (PDS), poly(orthoesters), polyhydroxyalkanates, polypeptides) or copolymers thereof.

In accordance with its purpose of use, the header (6) is manufactured from the same material as the body (2) or preferably from a magnetic metal. During the operation, after the body (2) of the biomaterial (1) is fixed to the defect area (11) till its last groove (3) the header (6) is removed from its place by turning it on the opposite direction.

Said header (6) part produces the force which will push the body (2) during compression. However, when the header (6) is being removed, it doesn't exceed the load which would move the body (2) since the body (2) has bigger grooves (3) having a larger holding force, by this way while turning the header (6) on the opposite direction the body (2) part stands still and the header (6) is removed.

After the header (6) part is removed, the blood coming from the bone marrow fills into the felt (7) part. After blood build up, the blood in the felt (7) part coagulates and provides a suitable framework for the growth factors and the stem cells to hold onto the medium and for formation of the desired tissue under the influence of micro environment.

The felt (7) part is prepared from materials selected from biodegradable polymers, animal-sourced biomaterials, glucoseamino glycanes, collagen, hyaluronic acid and derivatives, chitin, chitosan, alginate, gelatin, angyogenesis agents, morphogenic agents, anti - inflammatory agents or combinations thereof.

When more than one material (1) is placed into the defect area (11) the sizes of the felts (7) in this area may be different. These felts (7) can be trimmed by the applicant by using a suitable device such that they will be at the same height with the cartilage tissue (9) surrounding them. Therefore the height of the felt (7) part is more than the normal cartilage tissue (9). However, this trimming can be performed until the upper part of the body (2) emerged after removal of the header (6) part stays a few millimeters below this area.

The biomaterial (1) of the invention can be used solely for renewal of the bone tissue (10) and bone fixation. In this application, the felt (7) part is manufactured from the same material as the body (2).

Said scaffold (8) is prepared from α - tricalciumphospate (α - TCP), β - tricalciumphosphate (β - TCP), bone marrow aspirate (BMA), demineralized bone matrix (DBM), bone morphogenic protein (BMP), Hydroxyapatite (HA), calciumphosphate cement, collagen, hylaunoric acid and derivatives or combinations thereof which will allow new bone tissue (10) formation. In an embodiment of the invention, the controlled medicament for both cartilage tissue (9) and the bone tissue (10) may comprise systems that release cytokine and growth factor.

The biomaterial of the invention (1) is manufactured in diameters and lengths in accordance with the guiding channels known in the art. To cover bigger defect areas (11), it is implemented by arthroscopic or open surgery methods with multiple closest vicinity and with different diameters.

In another implementation said biomaterial (1) may be used for autolog condrocyte transplantation or implantation. In this embodiment, healthy cells taken from the patient are inocculated into the space (4) on the body (2) by a tissue engineering approach, the cells are proliferated in a suitable cell culture medium and then they are transferred into the defect area (11). Thus, bone cells are simultaneously grown in the space (4) of the body (4).

The main advantage of the biomaterial (1) of the present invention compared to similar materials existing in the current technology is that it can be implemented by arthroscopy without requiring open surgical operations. Thus, it is possible to avoid various disadvantages such as risks associated with anesthesia, long term treatment and costs which come along with the open surgery. Due to its geometric form and easy implementation, it is used in bigger defect areas (11). Due to the header (6) part which can be easily removed during the operation, it is provided that the blood coming from the bone marrow and thus the cells and the growth factors are transferred into the medium and they hold on to the medium thanks to the felt (7) part which acts as a scaffold. Therefore, the integration of the biomaterial (1) with the surrounding tissue is raised to a higher level, the vascularization rate is increased and tissue renewal is accelerated. Owing to the space(4) part and the channels (5) in the biomaterial (1), surface area in contact with the surrounding tissue is increased, and this in particular leads to an accelerated degradation rate of the material when compared to applications known in the art. Moreover, said structures enable the nutrition materials and the gasses to be transferred into the medium and enable the toxic materials and metabolites resulting from degredation to be removed from the medium, thus they protect the health of the patient by deceasing the risk of necrosis.

About this main concept, it is possible to develop various applications of the biomaterial (1) of the present invention and the invention cannot be limited with the examples described here and it is defined in the claims.

## Claims

1. A biomaterial (1) for use in the treatment of osteochondral bone defects and preferably in treatment of defects of bone covered by hyaline cartilage **comprising,**
- a body (2) manufactured from a biocompatible material,
- grooves (3) on the body (2) which provides advancing in the defect area,
- a space (4) in the body (2) which increases the contact area with the surrounding tissue,
- at least one channel (5) which opens to said space (4) and which enables the blood to be transferred into the medium and enables the toxic materials to be removed from the medium,
**and characterized by;**
- a felt (7) part which is affixed to the upper part of the body (2) by using biocompatible glues known in the art,
- a header (6) part located on the body (2) which can be removed after the material (1) is placed into the defect area (11) in the body (2).

2. A biomaterial (1) according to Claim 1 **characterized by** a body (2) which is manufactured from materials selected from alpha hydroxy acids (polyglycolide (PGA), poly(L - lactate), poly(D, L - lactate), poly(ε - caprolactone), poly(trimethylene carbonate), poly(ethylene oxide) (PEO), polyhydroxy butyrate (PHA), polo(β - hydroxybutyrate) (PHB), poly(β - hydroxyvalerate) (PHVA), poly(p-dioxane) (PDS), poly(orthoesters), polyhydroxyalkanates, polypeptides) or copolymers thereof.

3. A biomaterial (1) according to any of the previous claims **characterized by** a header (6) part which is manufactured from the same material as the body (2) or preferably from a magnetic material.

4. A biomaterial (1) according to any of the previous claims **characterized by** a felt (7) part which is prepared from materials selected from biodegradable polymers, biomaterials based on animals, glucoseamino glycanes, collagen, hyaluronic acid and derivatives, chitin, chitosan, alginate, gelatin, angyogenesis agents, morphogenic agents, anti - inflammatory agents or combinations thereof.

5. A biomaterial (1) according to any of the previous claims **characterized by a** scaffold (8) which is prepared from α - tricalciumphospate (α - TCP), β - tricalciumphosphate (β - TCP), bone marrow aspirate (BMA), demineralized bone matrix (DBM), bone morphogenic protein (BMP), Hydroxyapatite (HA), calciumphosphate cement, collagen, hylaunoric acid and derivatives or combinations thereof.

6. A biomaterial (1) according any of the previous claims **characterized by** a space (4) on the body (2) into which healthy cells taken from the patient are inocculated by a tissue engineering approach and the cells are proliferated in a suitable cell culture medium, prior to the implantation of the body (2) into the defect area (11).

## Patentansprüche

1. Ein Biomaterial (1) zur Anwendung bei der Behandlung von osteochondral Knochenschäden und vorzüglich bei der Behandlung von Knochenschäden, die durch glasartigen Knorpel bedeckt sind, **umfassend,**
- ein Gehäuse (2), das aus einem biologisch verträglichen Material hergestellt ist,
- Rillen (3) auf dem Gehäuse (2), welche das Fortschreiten im beschädigten Bereich ermöglichen,
- einen Raum (4) im Gehäuse (2), welcher den Kontaktbereich mit dem umgebenden Gewebe erhöht,
- zumindest einen Kanal (), welcher in den erwähnten Raum (4) einmündet und es ermöglicht, dass das Blut in das Medium übertragen und die toxischen Materialien aus dem Medium entfernt werden,
**und gekennzeichnet durch**
- einen Filzteil (7), welcher **durch** Anwendung von biologisch verträglichen, in der Technik bekannten Klebstoffen am oberen Teil des Gehäuses (2) befestigt ist,
- einen an dem Gehäuse (2) angeordneten Kopfteil (6), welcher abgebaut werden kann, nachdem das Material (1) im beschädigten Bereich (11) im Gehäuse (2) angeordnet ist.

2. Ein Biomaterial (1) nach Anspruch 1 **gekennzeichnet durch** ein Gehäuse (2), welches hergestellt ist aus Materialien, die aus der Gruppe bestehend aus Alpha-Hydroxysäuren (Poliglykolide (PGA), Poly(L-Laktat), Poly(D, L-Laktat), Poly(ε -Kaprolakton), Poly(Trimethylen Karbonat), Poly(Äthylen Oxid) (PEO), Polyhydroxy Butyrat (PHA), Poly(β-Hydroxybutyrat) (PHB), Poly(β-Hydroxyvalerat) (PHVA), Poly(P-Dioxan) (PDS), Poly(Orthoester), Polyhydroxyalkanate, Polypeptide) oder aus Kopolymeren davon gewählt sind.

3. Ein Biomaterial (1) nach einem der vorangehenden Ansprüche **gekennzeichnet durch** einen Kopfteil (6), welcher aus dem gleichen Material wie das Gehäuse (2) bzw. aus einem magnetischen Material hergestellt ist.

4. Ein Biomaterial (1) nach einem der vorangehenden Ansprüche **gekennzeichnet durch** einen Filzteil (7), welcher aus Materialien hergestellt ist, die aus biologisch abbaubaren Polymeren, aus den auf Tieren basierenden Biomaterialien, Glukose-Amino Glykanen, Kollagenen, Hyaluronsäuren und Derivaten, Chitin, Chitosan, Alginat, Gelatine, Angiogenesis-Mittel, morphogenischen Mittel, entzündungshemmende Mittel oder Kombinationen davon bestehen.

5. Ein Biomaterial (1) nach einem der vorangehenden Ansprüche **gekennzeichnet durch** ein Gerüst (8), welches aus α -Trikalziumphosphat (α-TCP), β-Trikalziumphosphat (β-TCP), Knochenmark-Aspirata (BMA), entsalztes Knochen-Bindemittel (DBM), Knochen-Morphogenisches Eiweiß (BMP), Hydroxyapatit (HA), Kalziumphosphat-Bindemittel, Kollagenen, Hyaluronsäure und Derivaten oder asu Kombinationen davon vorbereitet ist.

6. Ein Biomaterial (1) nach einem der vorangehenden Ansprüche **gekennzeichnet durch** einen Raum (4) auf dem Gehäuse (2), in den die von dem Patienten entnommenen gesunden Zellen **durch** eine Gewebezühtungsmethode geimpft werden und wobei sich die Zellen in einem geeigneten Zellkultur-Medium vor der Einpflanzung des Gehäuses (2) in den beschädigten Bereich (11) vermehren.

## Revendications

1. Un biomatériau pour l'usage dans le traitement des anomalies ostéochondrales d'os et de préférence dans le traitement des anomalies d'os recouvert de cartilage hyalin, **comportant:**
- un corps (2) fabriqué d'un matériau biocompatible,
- les rainures (3) sur le corps (2) qui garantissent l'avancement dans la zone défectueuse,
- un espace (4) à l'intérieur du corps (2) qui augment la zone de contact avec le tissu environnant,
- au moins un canal (5) aboutit dans ledit espace (4) et permettant au sang d'être transféré dans le milieu et permettant aux matériaux toxiques d'être élimine du milieu,
**et caractérisé par:**
- une partie de feutre (7) qui est apposée sur la partie supérieure du corps (2) en utilisant des colles biocompatibles connues dans l'art,
- une partie de tête (6) placée sur le corps (2) qui peut être détachée après le placement du matériau à l'intérieur de la zone défectueuse (11) dans le corps (2).

2. Un matériau biocompatible selon la revendication 1, **caractérisé par** un corps (2) produit des matériaux sélectionnés parmi les acides alpha hydroxy (polglycolide (PGA), ' poly(L - lactate), poly(D, L - lactate), poly(e - caprolactone), poly(triméthylène carbonate), poly(éthylène oxyde) (PEO), polyhydroxy butyrate (PHA), poly(p - hydroxybutyrate) (PHB), poly(P - hydroxyvalerate) (PHVA), poly(p-dioxane) (PDS), poly(orthoesters), polyhydroxyalkanates, polypeptides) ou leurs copolymères.

3. Un **biomatériau** (1) selon l'une des revendications précédentes, **caractérisé par** une partie de tête (6) produit du même matériau du corps (2) ou de préférence d,'un matériau magnétique.

4. Un biomatériau (1) selon l'un des revendications précédentes, **caractérisé par une** partie de feutre (7) qui est préparé à partir des matériaux sélectionnés parmi les polymères biodégradables, les biomatériaux fondés sur les animaux, les glycanes glucoseamines, le collagène, l'acide hyaluronique et ses dérivatives, la chitine, le chitosan, la gélatine, les agents angiogèneses, les agents morphogeniques, les agents anti-inflammatoires, ou leurs combinaisons.

5. Un biomatériau (1) selon l'un des revendications précédentes, **caractérisé par** un échafaud (8) qui est préparé à partir d'**α** - phosphate tricalcique (α - TCP), β - phosphate tricalcique (β - TCP), l'aspirât de moelle osseuse (AMO), la matrice d'os déminéralisé (MOD), la protéine morphogénique osseuse (PMO), l'hydroxyapatite (HA), le ciment à base de phosphate de calcium, le collagène, l'acide hyaluronique et ses dérivatives ou leurs combinaisons.

6. Un biomatériau (1) selon l'un des revendications précédentes, **caractérisé par** un espace (49 sur le corps 2) dans lequel on inocule les cellules saines prises du patient par une approche de l'ingénierie tissulaire et on prolifère les cellules dans un moyen de culture cellulaire convenable avant de l'implantation du corps (2) à l'intérieur de la zone défectueuse (11).
